# EUROPÄISCHE PATENTANMELDUNG

(11) **EP 4 002 762 A1**
(43) Veröffentlichungstag der Anmeldung: **25.05.2022**
(21) Anmeldenummer: 21020554.8
(22) Anmeldetag: 08.11.2021
(51) Int. Cl.: H04L 9/40, H04L 9/32, A61M 16/00

(54) **AUTHENTIFIZIERUNG EINES BEATMUNGSGERÄTES**

(30) Priorität: 19.11.2020 DE 102020007090
(71) Anmelder: Löwenstein Medical Technology S.A., 2557 Luxembourg (LU)
(72) Erfinder: Bychkov, Igor, 76275 Ettlingen (DE); Schwaibold, Matthias, 76228 Karlsruhe (DE)
(74) Vertreter: Marx, Thomas

(57) **Zusammenfassung**

Verfahren zur Authentifizierung zumindest eines Beatmungsgerätes bei zumindest einer Gegenstelle wobei sich das Beatmungsgerät über zumindest eine Schnittstelle mit der Gegenstelle verbinden kann, wobei auf dem Beatmungsgerät mindestens eine Authentifizierungsdatei gespeichert ist, wobei die Authentifizierungsdatei zumindest einen Signaturcode einer Signiereinrichtung beinhaltet und der Gegenstelle ein öffentlicher Schlüsselcode der Signiereinrichtung bekannt ist, wobei das Beatmungsgerät bei Verbindungsaufbau mit der Gegenstelle die Authentifizierungsdatei an die Gegenstelle sendet, wobei die Gegenstelle mit dem öffentlichen Schlüsselcode den Signaturcode der Authentifizierungsdatei daraufhin überprüft, ob der Signaturcode von der Signierstelle stammt und wobei das Beatmungsgerät authentifiziert wird, wenn die Gegenstelle den Signaturcode als von der Signiereinrichtung stammend erkennt.

## Beschreibung

Die Erfindung betrifft ein Verfahren zur Authentifizierung eines Beatmungsgerätes gegenüber Gegenstellen.

Bei der Datenübertragung zwischen Beatmungsgerät und Servern, wie bei der Fernüberwachung oder der Telemedizin, werden zum Teil hochsensible Daten übertragen, welche unter anderem Daten zum Gesundheitszustand und der Therapie eines Patienten beinhalten können. Essentiell wichtig ist es dabei, dass den Beatmungsgeräten bei der Datenübertragung auch ein entsprechender Anwender zugeordnet wird. Aus dem Stand der Technik sind dazu einige Identifizierungsverfahren bekannt, welche zwar eine Identifizierung des Beatmungsgerätes erlauben, eine sichere Überprüfung der Echtheit der Identität ist dabei nicht gegeben, sodass sich auch andere Datenquellen als das Beatmungsgerät ausgeben können und so beispielsweise falsche Daten übermitteln.

Ist eine der Hardwarekomponenten, insbesondere spezielle Komponenten, welche speziell konfiguriert sind, defekt, kann dies zu weiteren Problemen hinsichtlich der Identifizierung führen, insbesondere da durch die defekte Hardwarekomponente auch Identifizierungsmerkmale verloren gehen.

Aufgabe der vorliegenden Erfindung ist daher die Bereitstellung eines Verfahrens zur sicheren Datenübertragung eines Beatmungsgeräts. Die Aufgabe wird durch ein Verfahren nach Anspruch 1 sowie durch die Vorrichtung nach Anspruch 23 gelöst.

Verfahren zur Authentifizierung zumindest eines Beatmungsgerätes bei zumindest einer Gegenstelle wobei sich das Beatmungsgerät über zumindest eine Schnittstelle mit der Gegenstelle verbinden kann, wobei auf dem Beatmungsgerät mindestens eine Authentifizierungsdatei gespeichert ist, wobei die Authentifizierungsdatei zumindest einen Signaturcode einer Signiereinrichtung beinhaltet und der Gegenstelle ein öffentlicher Schlüsselcode der Signiereinrichtung bekannt ist, wobei das Beatmungsgerät bei Verbindungsaufbau mit der Gegenstelle die Authentifizierungsdatei an die Gegenstelle sendet, wobei die Gegenstelle mit dem öffentlichen Schlüsselcode den Signaturcode der Authentifizierungsdatei daraufhin überprüft, ob der Signaturcode von der Signierstelle stammt und wobei das Beatmungsgerät authentifiziert wird, wenn die Gegenstelle den Signaturcode als von der Signiereinrichtung stammend erkennt.

In manchen Ausführungsformen des Verfahrens werden nach und/oder während des Verbindungsaufbaus therapeutische Daten vom Beatmungsgerät auf die Gegenstelle übertragen, wobei die therapeutischen Daten von der Gegenstelle nur dann verarbeitet werden, wenn das Beatmungsgerät authentifiziert wird. Somit können also parallel zur Authentifizierungsdatei bereits therapeutische Daten übertragen werden. Diese Daten werden aber erst dann verarbeitet, wenn die Authentifizierung des Beatmungsgerätes durchgeführt ist.

In manchen Ausführungsformen des Verfahrens wird die Verbindung vom Beatmungsgerät zur Gegenstelle nur dann aufgebaut und/oder akzeptiert und/oder aufrechterhalten, wenn die Gegenstelle den Signaturcode als von der Signiereinrichtung stammend erkennt und das Beatmungsgerät authentifiziert ist.

In manchen Ausführungsformen des Verfahrens werden vom Beatmungsgerät an die Gegenstelle übertragene therapeutische Daten von der Gegenstelle verworfen, wenn die Gegenstelle den Signaturcode nicht als von der Signiereinrichtung stammend erkennt.

In manchen Ausführungsformen des Verfahrens erstellt die Signiereinrichtung die Authentifizierungsdatei und fügt der Authentifizierungsdatei unter Verwendung eines geheimen Schlüssels der Signiereinrichtung den Signaturcode hinzu.

In manchen Ausführungsformen des Verfahrens beinhaltet die Authentifizierungsdatei neben dem Signaturcode mindestens eine Identifikationsnummer des Beatmungsgerätes, wobei die Gegenstelle das Beatmungsgerät anhand der Identifikationsnummer erkennt und einem Anwender zuordnet.

In manchen Ausführungsformen des Verfahrens erzeugt das Beatmungsgerät eine Anfragedatei zur Anfrage der Erstellung einer Authentifizierungsdatei durch die Signiereinrichtung, wobei die Anfragedatei mindestens die Identifikationsnummer des Beatmungsgerätes enthält.

In manchen Ausführungsformen des Verfahrens erstellt das Beatmungsgerät neben der Anfragedatei eine Informationsdatei, welche mindestens die Identifikationsnummer des Beatmungsgerätes und mindestens eine Identifikationsnummer mindestens einer Hardwarekomponente des Beatmungsgerätes enthält, wobei die Anfragedatei und die Informationsdatei auf eine Prüfeinrichtung übertragen werden, die in der Informationsdatei enthaltene Kombination der Identifikationsnummern auf Gültigkeit überprüft wird und wenn die Kombination der Identifikationsnummern gültig ist, die Prüfeinrichtung die Anfragedatei an die Signiereinrichtung weiterleitet und die Signiereinrichtung unter Verwendung der Anfragedatei die Authentifizierungsdatei erzeugt.

In manchen Ausführungsformen des Verfahrens wird die übertragene Kombination der Identifikationsnummern in eine Datenbank eingetragen, falls mit der Identifikationsnummer des Beatmungsgerätes noch keine Kombination mit einer Identifikationsnummer einer Hardwarekomponente eingetragen ist. Falls eine Kombination der Identifikationsnummer des Beatmungsgerätes mit der Identifikationsnummer einer Hardwarekomponente eingetragen ist, welche nicht der übertragenen Kombination der Identifikationsnummern entspricht, wird die übertragene Kombination als nicht gültig gewertet und die Anfragedatei wird nicht an die Signiereinrichtung weitergeleitet.

In manchen Ausführungsformen des Verfahrens wird bei Austausch der Hardwarekomponente eine neue Anfragedatei vom Beatmungsgerät unter Verwendung zumindest einer Identifikationsnummer der neuen Hardwarekomponente und der Identifikationsnummer des Beatmungsgerätes erstellt.

In manchen Ausführungsformen des Verfahrens wird die neue Kombination von Identifikationsnummer der neuen Hardwarekomponente und Identifikationsnummer des Beatmungsgerätes auf Gültigkeit überprüft, wobei bei einer gültigen neuen Kombination der Identifikationsnummern die Anfragedatei auf die Signiereinrichtung übertragen wird, wobei die Signiereinrichtung eine neue Authentifizierungsdatei erstellt und unter Verwendung des geheimen Schlüsselcodes der neuen Authentifizierungsdatei einen Signaturcode hinzufügt und die neue Authentifizierungsdatei danach auf das Beatmungsgerät übertragen wird.

In manchen Ausführungsformen des Verfahrens wird die neue Kombination von Beatmungsgerät und neuer Hardwarekomponente beziehungsweise deren Identifikationsnummern direkt in der Signiereinrichtung überprüft.

In manchen Ausführungsformen des Verfahrens wird die alte Kombination der Identifikationsnummern des Beatmungsgerätes und der alten Hardwarekomponente nach Tausch der alten Hardwarekomponente mit der neuen Hardwarekomponente als ungültig markiert.

In manchen Ausführungsformen des Verfahrens werden als ungültig markierte Kombinationen von Beatmungsgeräten und Hardwarekomponenten der Gegenstelle zur Verfügung gestellt.

In manchen Ausführungsformen des Verfahrens ruft die Gegenstelle zur Verfügung gestellten und als ungültig markierten Kombinationen von Zeit zu Zeit ab, wobei die Gegenstelle eine Verbindung mit einem Beatmungsgerät welches sich mit einer Authentifizierungsdatei authentifizieren möchte, welche unter Verwendung einer ungültigen Kombination erstellt wurde, nicht akzeptiert, sodass keine Verbindung zwischen dem Beatmungsgerät und der Gegenstelle aufgebaut wird.

In manchen Ausführungsformen des Verfahrens ruft die Gegenstelle zur Verfügung gestellten und als ungültig markierten Kombinationen von Zeit zu Zeit ab, wobei die Gegenstelle eine Verbindung mit einem Beatmungsgerät welches sich mit einer Authentifizierungsdatei authentifizieren möchte, welche unter Verwendung einer ungültigen Kombination erstellt wurde, nicht akzeptiert, sodass keine Übertragung von therapeutischen Daten zwischen dem Beatmungsgerät und der Gegenstelle stattfindet und/oder die vom Beatmungsgerät an die Gegenstelle gesendeten therapeutischen Daten nicht von der Gegenstelle verarbeitet werden und/oder von der Gegenstelle verworfen werden.

In manchen Ausführungsformen des Verfahrens generiert die Gegenstelle bei einem Authentifizierungsversuch eines Beatmungsgerätes mit einer Authentifizierungsdatei aus einer ungültigen Kombination von Hardwarekomponente und Beatmungsgerät eine Meldung.

In manchen Ausführungsformen des Verfahrens sendet die Gegenstelle bei einem Authentifizierungsversuch eines Beatmungsgerätes mit einer Authentifizierungsdatei aus einer ungültigen Kombination einen Befehl an das Beatmungsgerät, welcher die Benutzung des Beatmungsgerätes sperrt und eine Servicemeldung auf einer Anzeigeeinrichtung des Beatmungsgerätes anzeigt.

In manchen Ausführungsformen des Verfahrens wird das von der Hardwarekomponente ein geheimer Schlüsselcode und ein öffentlicher Schlüsselcode für das Beatmungsgerät generiert und der öffentliche Schlüsselcode als Teil der Anfragedatei an die Signiereinrichtung übermittelt, wobei der öffentliche Schlüsselcode zur Verschlüsselung von Daten dient, welche an das Beatmungsgerät gesendet werden und die mit dem öffentlichen Schlüsselcode des Beatmungsgerätes verschlüsselten Daten nur mit den geheimen Schlüsselcode des Beatmungsgerätes entschlüsselt werden können.

In manchen Ausführungsformen des Verfahrens verfügt die Gegenstelle über einen geheimen Schlüsselcode und einen öffentlichen Schlüsselcode, wobei der öffentliche Schlüsselcode als Teil einer Authentifizierungsdatei dem Beatmungsgerät zur Verfügung gestellt wird, wobei das Beatmungsgerät Daten, welche vom Beatmungsgerät an die Gegenstelle übertragen werden, mit dem öffentlichen Schlüsselcode verschlüsseln und wobei die Daten, welche mit dem öffentlichen Schlüsselcode verschlüsselt wurden, nur mit dem geheimen Schlüsselcode entschlüsselt werden können.

In manchen Ausführungsformen des Verfahrens wird die Authentifizierungsdatei von der Signiereinrichtung erstellt und die Signiereinrichtung fügt der Authentifizierungsdatei einen Signaturcode unter Verwendung des geheimen Schlüsselcodes hinzu, wobei das Beatmungsgerät den Signaturcode mit dem öffentlichen Schlüsselcode überprüft und die Authentifizierungsdatei nur akzeptiert, wenn der Signaturcode als von der Signiereinrichtung stammend erkannt wird.

In manchen Ausführungsformen des Verfahrens ist die Hardwarekomponente ein Mainboard.

System umfassend zumindest ein Beatmungsgerät und zumindest eine Gegenstelle wobei das Beatmungsgerät eingerichtet ist über zumindest eine Schnittstelle eine Verbindung mit der Gegenstelle aufzubauen und wobei auf dem Beatmungsgerät zumindest eine Authentifizierungsdatei gespeichert ist, welche zumindest einen Signaturcode einer Signiereinrichtung beinhaltet, wobei der Gegenstelle ein öffentlicher Schlüsselcode der Signiereinrichtung bekannt ist, wobei das Beatmungsgerät bei Verbindungsaufbau mit der Gegenstelle die Authentifizierungsdatei an die Gegenstelle sendet, wobei die Gegenstelle mit dem öffentlichen Schlüsselcode den Signaturcode der Authentifizierungsdatei daraufhin überprüft, ob der Signaturcode von der Signierstelle stammt und wobei therapeutische Daten nur dann übertragen und/oder verarbeitet werden und/oder die Verbindung aufgebaut und/oder akzeptiert wird, wenn die Gegenstelle den Signaturcode als von der Signiereinrichtung stammend erkennt.

Das System ist dazu eingerichtet und ausgebildet, das beschriebene Verfahren und/oder einzelne Verfahrensschritte durchzuführen.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter Beatmungsgeräten können Geräte in Form von Beatmungsgeräten und Diagnosegeräten verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können. Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte (teilweise bekannt als BiPAP), Narkose- bzw. Anästhesiegeräte, Atemtherapiegeräte, (klinische, außerklinische oder Notfall-) Beatmungsgeräte, Highflow-Therapiegeräte und Hustenmaschinen.

Eine Schnittstelle ist im weitesten Sinne als jede Art von Einrichtung zur Verbindung - unabhängig der Verbindungsart - zwischen dem Beatmungsgerät und einer Gegenstelle oder einer Person zu verstehen. Eine Schnittstelle für eine Verbindung zwischen dem Beatmungsgerät und einer Person kann dabei zum Beispiel eine Benutzerschnittstelle sein, welche die Interaktion zwischen Person und Beatmungsgerät direkt am Beatmungsgerät ermöglicht. Weitere Schnittstellen können beispielsweise Schnittstellen zur drahtlosen Verbindung über kurze (z.B. Bluetooth, WPAN, etc.) und/oder lange Entfernungen (Mobilfunk, UMTS, 3G, LTE, 5G, WiFi, WLAN, etc.), Schnittstellen für kabelgebundene Verbindungen (unter anderem LAN, USB-Verbindungen, direkte Verbindungen mit Computern, Sensoren, Diagnose- und Medizintechnikgeräten) und auch Steckverbindungen (Steckplatz für SD-Karten, USB-Anschluss, etc.) sein.

Die einzelnen Bestandteile des Beatmungsgeräts können in Module unterteilt sein, wobei auch das gesamte Beatmungsgerät als ein einziges Modul verstanden werden kann. Die Definition eines Moduls kann unter verschiedenen Gesichtspunkten erfolgen, welche an dieser Stelle nicht näher erläutert werden und keinen Einfluss auf die Erfindung als solche haben. Auch ein Überschneiden von verschiedenen Modulen durch die Bestandteile des Beatmungsgeräts ist dabei möglich. So kann ein Bestandteil beispielsweise zu zwei Modulen zugeordnet sein. Weiter können die einzelnen Module auch zu Modulgruppen zusammengefasst werde, wobei auch diese Modulgruppen unter Umständen Überschneidungen aufweisen können.

Die Quelle einer Dateneingabe kann verschiedene Formen haben, resultiert aber in der Regel in einem elektrischen Signal, welches innerhalb des Beatmungsgeräts verarbeitet werden kann. Beispielsweise kann die Dateneingabe mechanisch über eine Benutzerschnittstelle in Form eines berührungssensitiven Bildschirms erfolgen. Über den berührungssensitiven Bildschirm wird die mechanische Berührung in ein elektrisches Signal umgewandelt, welches im Beatmungsgerät verarbeitet werden kann. Über andere Schnittstellen kann beispielsweise aber auch direkt ein elektrisches Signal eingegeben werden. An dieser Stelle wird von einer genauen Beischreibung aller möglichen Wege einer Dateneingabe am Beatmungsgerät zu tätigen bzw. über eine Schnittstelle zu übermitteln verzichtet, da die Form der Dateneingabe keine Relevanz für das erfinderische Verfahren bzw. die erfinderische Vorrichtung hat.

Bei der Handhabung der Authentifizierung erhält das Beatmungsgerät ein Zertifikat, welches zur Authentifizierung des Beatmungsgerätes und optional auch zur Verschlüsselung von Daten mit einem privaten Schlüssel dient. Hierzu wird beispielsweise eine entsprechende Authentifizierungsdatei, letztlich also ein Zertifikat, für das Beatmungsgerät erstellt und entsprechend signiert. Die Signierung, beispielweise durch Hinzufügen eines entsprechenden Signaturcodes, wird in der Regel durch eine vertrauenswürdige Signiereinrichtung (Certification Authority, CA) durchgeführt. Die Authentifizierung ist beispielsweise nötig, damit sichergestellt werden kann, dass die vom Beatmungsgerät an die Gegenstelle übermittelten Daten auch tatsächlich von dem Beatmungsgerät stammen und nicht vorgetäuscht wird, dass das Beatmungsgerät die Daten schickt.

Während der Herstellung des Beatmungsgerätes wird dabei eine Kombination unter anderem aus der Identifikationsnummer des Beatmungsgerätes (zum Beispiel die Seriennummer des Beatmungsgerätes), einer Identifikationsnummer einer Hardwarekomponente und einem Credential (beispielsweise ein Signaturcode oder einem öffentlichen Schlüsselcode (Public Key)) generiert beziehungsweise definiert und in einer Datenbank gespeichert. Die Identifikationsnummer einer Hardwarekomponente kann beispielsweise die Seriennummer des Mainboards sein und/oder jede andere individuelle Nummer/Bezeichnung einer Komponente wie beispielsweise Seriennummern eines einzelnen Chips. Die Gültigkeit dieser Kombination kann so zu jedem späteren Zeitpunkt wieder überprüft werden.

Ist die Hardwarekomponente, deren Identifikationsnummer zur Erstellung der Authentifizierung verwendet wurde, defekt und muss getauscht werden, entsteht zunächst eine ungültige Kombination, da bei Herstellung der Hardwarekomponente (zum Beispiel Mainboard) noch nicht bekannt war, in welches Beatmungsgerät mit welcher Identifikationsnummer (zum Beispiel Seriennummer) die Hardwarekomponente später verbaut wird. Auch der Credential/die Schlüsselcodes (Public und Private Key) für die Kombination ist noch nicht erstellt. Daher folgen dem Austausch zum Beispiel folgende Schritte: Zunächst wird die alte Kombination durch Angabe von Identifikationsnummer des Beatmungsgerätes und der Hardwarekomponenten aus der Datenbank ausgetragen. Dadurch wird gleichzeitig der Tausch der Hardwarekomponenten freigegeben. Die Identifikationsnummer der neuen Hardwarekomponenten sowie des Beatmungsgerätes wird dann in das Beatmungsgerät über eine Schnittstelle (z.B. Tastatur, Touchscreen, etc.) eingegeben. Über eine (andere) Schnittstelle kann dann eine Kommunikation der Kombination an eine Gegenstelle, z.B. einen (Credential-)Server, erfolgen, sodass ein neuer Credential für die Kombination erstellt wird und/oder ein entsprechendes Zertifikat erstellt/signiert wird. Auch wird die neue Kombination in der Datenbank abgelegt.

Sollte zudem durch zum Beispiel ein Datensicherheits-Leck die eigentlich geheimen Credentials öffentlich werden, so kann über diesen Weg auch ein neuer, geheimer/privater Credential erstellt werden.

Jedes Beatmungsgerät bekommt während der Herstellung ein persönliches Zertifikat (Authentifizierungsdatei), welches unter anderem die Geräte-Seriennummer (Identifikationsnummer des Beatmungsgerätes) enthält. Mit diesem Zertifikat ist es später möglich, dass das Beatmungsgerät identifiziert und auch authentifiziert werden kann - z.B. während der Online-Kommunikation oder der Validierung der Therapie-Daten.

Die dazu nötigen Schritte werden von einem Prüfstands-PC (Prüfeinrichtung) durchgeführt. Das Zertifikat wird wie folgt erstellt:
1. Das Beatmungsgerät bekommt Informationen über die Geräte-Seriennummer gesetzt
2. Das Beatmungsgerät generiert selbst ein Schlüsselpaar uns speichert den privaten Schlüssel so dass dieser nie ausgelesen werden kann
3. Das Beatmungsgerät erstellt daraus eine Zertifikatsanfrage (CSR, Certificate Singning Request)
4. Die Prüfeinrichtung empfängt die Anfrage und sendet diese Anfrage an den PKI-Server
5. Der PKI-Server erstellt das Zertifikat basierend auf der Anfrage
6. Der PC importiert/überträgt das Zertifikat auf das Beatmungsgerät

Dem PKI-Server werden zusätzliche Informationen über einzelne im Geräte verbaute Komponenten zur Verfügung gestellt

Mit der Hilfe von diesem Zertifikat und dem gespeicherten Schlüsselpaar kann sich das Gerät unter Benutzung verschiedener moderner Technologien authenzifizieren.

Im Folgenden wird kurz der Austausch einer Hardwarekomponente, deren Identifikationsnummer zur Generierung der Authentifizierungsdatei verwendet wurde, am Beispiel des Mainboards dargestellt, wobei der Vorgang auch auf andere austauschbare Komponenten übertragbar ist.

Nach Ersetzen des Mainboards sollte die Geräte-Seriennummer nicht geändert werden, da unter anderem die Seriennummer häufig verwendet wird, um Patienten zu Beatmungsgeräten zuzuordnen. Der Tausch des Mainboards kann außerhalb der Produktionsstätte stattfinden, beispielsweise bei einem Zwischenhändler oder Dienstleister, welcher keine vertrauenswürdigen Zertifikate ausstellen oder keine vertrauenswürdige Verbindung mit der PKI des Herstellers haben kann.

Daher wurde ein Verfahren entwickelt, welches die Möglichkeit eröffnet, dass der Kunde oder Händler die ursprüngliche Seriennummer wiederverwenden kann, nachdem das Mainboard ersetzt wurde und ein neues Zertifikat vom Hersteller erhalten kann, ohne, dass spezielle Software oder Ausrüstung für den Kunden/Händler notwendig sind.

Mainboards, die als Austausch-Mainboards verwendet werden, werden gesondert als Ersatzteile produziert und durchlaufen einen besonderen finalen Prozess. Während der Produktion des Ersatzmainboards bekommt der PKI-Service bereits Informationen über die produzierten Ersatzmainboards. Der PKI-Service kann ein Server sein, welcher alles automatisch prozessiert, kann aber auch Personen umfassen, welche einzelne Schritte durchführen.

Während der Produktion teilt ein Initialisierungs-PC/eine Prüfeinrichtung dem PKI-Service den Public Key (öffentlicher Schlüsselcode) und die Seriennummer (Identifikationsnummer) des Ersatzmainboards mit. Folgende Schritte werden durchgeführt:

### Produktion der Ersatzmainboards

1. Die Prüfeinrichtung initialisiert das Ersatzmainboard und aktiviert die Firmware
2. Auf diesem Mainboard wird eine Software gestartet, die das Schlüsselpaar (privater und öffentlicher Schlüssel) erzeugt und Informationen über den Public Key und die Seriennummer von verschiedenen Maiborardkomponenten sowie dem Mainboard selbst der Prüfeinrichtung mitteilt. Dies kann z.B, über eine Datei (beispielsweise im JSON-Format) der Prüfeinrichtung passieren.
3. Die Prüfeinrichtung sendet diese Informationen an den PKI-Service.

### Austausch des Mainboards:

1. Das defekte Mainboard wird beispielsweise von einem Techniker ausgebaut und ein Ersatzmainboard wird eingebaut.
2. Das Beatmungsgerät wird gestartet und die (gleiche) Seriennummer des Beatmungsgerätes wird über eine Schnittstelle eingegeben.
3. Das Beatmungsgerät generiert eine Anfrage zur Erstellung/Signierung eines Zertifikates.
4. Die Seriennummer (Identifikationsnummer) des defekten Mainboards sowie von den einzelnen Komponenten des Mainboards wird zusammen mit der Zertifikats-Anfrage (der Anfragedatei) an den PKI-Service übermittelt. Beispielsweise per Email, kann aber auch vollautomatisch erfolgen.
5. Beim PKI-Service wird geprüft, ob die Seriennummer der Komponenten des alten Mainboards mit der Seriennummer des Beatmungsgerätes, welches ein neues Zertifikat erhalten soll, kombiniert war und ob der Public Key des verbauten Ersatzmainboards gültig ist und während der Produktion beim Hersteller erfasst wurde.
6. Das Zertifikat wird daraufhin basierend auf dem neuen CSR vom PKI-Service erstellt, signiert und in einer Datei gespeichert.
7. Die Datei wird an die Person, die das Mainboard austauscht, übertragen, und das Beatmungsgerät kann das neue Zertifikat über SD Karte auslesen und akzeptieren

Erfindungsgemäß kann auch vorgesehen sein, dass die Gegenstelle, welche mit dem Beatmungsgerät verbunden ist bzw. wird, ebenfalls über eine Authentifizierungsdatei verfügt, welch einen durch die Signierstelle erzeugten Signaturcode umfasst. Über diesen Signaturcode kann das Beatmungsgerät die Gegenstelle authentifizieren.

Auch kann die erfindungsgemäße Authentifizierung dazu dienen, beispielsweise Konfigurationsdaten für das Beatmungsgerät zu signieren und/oder zu verschlüsseln. Dabei werden von der Gegenstelle Konfigurationsdaten an das Beatmungsgerät übertragen. Die Konfigurationsdaten werden beispielsweise bei Erstellung von der Gegenstelle signiert. Über die Signatur (z.B. den Signaturcode) kann das Beatmungsgerät die Gültigkeit und/oder Integrität und/oder die Berechtigung der Konfigurationsdaten prüfen. Die Übertragung kann beispielsweise über eine der Schnittstellen online (Datenverbindung über Modem, Netzwerk, WIFI, Bluetooth, USB etc.) oder auch offline (Speichermedium, Datei, USB, etc.) erfolgen. In manchen Ausführungsformen kann vorgesehen sein, dass die Konfigurationsdaten von einer anderen Gegenstelle erstellt werden als jene, über welche die Konfigurationsdaten an das Beatmungsgerät übertragen werden. Beispielhaft können die Konfigurationsdaten an einem PC erstellt werden, welcher die Konfigurationsdaten mit dem Signaturcode des PCs signiert werden, wobei der Signaturcode des PCs von dem Beatmungsgerät überprüft werden kann um die Authentizität der Konfigurationsdaten zu überprüfen. Die Konfigurationsdaten werden dann beispielsweise über einen Server an das Beatmungsgerät übertragen, wobei sich der Server zusätzlich beim Beatmungsgerät authentifizieren muss und auch das Beatmungsgerät beim Server authentifiziert. Beispielsweise kann so sichergestellt werden, dass die Konfigurationsdaten an das richtige Beatmungsgerät übertragen werden. In manchen Ausführungsformen wird so auch sichergestellt, dass die Konfigurationsdaten nur an das bestimmte Beatmungsgerät übertragen werden.

Alternativ oder ergänzend kann auch vorgesehen sein, dass die Konfigurationsdaten mithilfe des öffentlichen Schlüssels des Beatmungsgerätes verschlüsselt werden. Beispielsweise kann so sichergestellt werden, dass die Konfigurationsdaten nur von einem einzigen Beatmungsgerät akzeptiert bzw. verwendet werden können.

Das erfindungsgemäße Verfahren kann auch auf Software-Module, z.B. einem Firmware-Update, übertragen werden. Die Software-Module werden dabei entsprechend signiert, sodass das Beatmungsgerät die Herkunft des Software-Moduls authentifizieren kann. In manchen Ausführungsformen ist optional vorgesehen, dass sich das Beatmungsgerät erfindungsgemäß bei der Gegenstelle authentifizieren muss, damit das Software-Modul und/oder Konfigurationsdaten übertragen werden. Beispielsweise kann so sichergestellt werden, dass die bereitgestellten Software-Module und/oder Konfigurationsdaten nur auf das bestimmte Beatmungsgerät übertragen werden.

Auch bei Software-Modulen, welche an das Beatmungsgerät übertragen werden sollen, kann vorgesehen sein, dass die Software-Module mit dem öffentlichen Schlüssel des Beatmungsgeräts verschlüsselt werden, sodass nur das bestimmte Beatmungsgerät die bereitgestellten Software-Module nutzen kann.

In manchen Ausführungsformen kann vorgesehen sein, dass das Beatmungsgerät Funktionen umfasst, welche optional gesperrt sind. Es kann dazu vorgesehen sein, dass diese Funktionen beispielsweise über Konfigurationsdaten freigeschaltet werden. Wie zuvor erläutert können diese Konfigurationsdaten entsprechend signiert und/oder verschlüsselt sein, sodass vom Beatmungsgerät geprüft werden kann, ob die Freischaltung erlaubt ist. Beispielsweise wird die Authentizität der Herkunft der Konfigurationsdaten überprüft. In manchen Ausführungsformen können die Konfigurationsdaten auch Daten zur Freischaltung eines Experten- und/oder Servicemodus enthalten. Ein Experten- und/oder Service-Modus erlaubt beispielsweise den Zugriff auf erweiterte Einstellungsmöglichkeiten, welche einem Anwender/Patienten nicht zur Verfügung stehen sollten.

In manchen Ausführungsformen können beispielsweise Konfigurationsdaten auf einem Datenträger enthalten sein, welche die Freischaltung des Experten- und/oder Servicemodus enthalten. Das Beatmungsgerät kann die Authentizität der Freischaltung entsprechend der Signatur überprüfen und den jeweiligen Modus freischalten. Es kann beispielsweise auch vorgesehen sein, dass der Modus nur solange aktiviert ist, wie der Datenträger mit dem Beatmungsgerät verbunden ist. Ähnliches kann auch für entfernte Gegenstellen gelten, der Experten- und/oder Service-Modus bleibt nur so lange aktiviert, wie die entfernte Gegenstelle mit dem Beatmungsgerät verbunden ist. Gegebenenfalls kann auch vorgesehen sein, dass über die entfernte Gegenstelle ein solcher Experten- und/oder Service-Modus aktiviert/deaktiviert werden kann, in manchen Ausführungsformen auch unabhängig vom Verbindungsstatus zwischen Gegenstelle und Beatmungsgerät.

In manchen Ausführungsformen kann auch vorgesehen sein, dass über das Zertifikat bzw. die Authentifizierungsdatei selbst eine Freischaltung und/oder Konfiguration von Modi erfolgen kann. Beispielsweise kann ein Beatmungsgerät die technischen Voraussetzungen für einen APAP-Betrieb mitbringen, ist aber zunächst als CPAP-Gerät konfiguriert. Die Authentifizierungsdatei kann dazu etwa neben einer oder mehrerer Identifikationsnummern auch eine Information enthalten, als welche Art von Beatmungsgerät das Gerät konfiguriert werden soll. Zum Beispiel erhält das Beatmungsgerät dazu ein aktualisiertes Zertifikat mit den neuen Informationen. Alternativ oder ergänzend kann auch vorgesehen sein, dass in dem Zertifikat bzw. der Authentifizierungsdatei, welche auf das Beatmungsgerät aufgespielt wird, ein entsprechender Freischaltungscode enthalten ist.

Das erfindungsgemäße Verfahren erlaubt unter anderem auch eine eindeutige Identifizierung des Beatmungsgerätes und eine gleichzeitige Authentifizierung. So kann beispielsweise auch sichergestellt werden, dass neben der Herkunft der Daten vom Beatmungsgerät auch Daten, welche ausschließlich für das Beatmungsgerät gedacht sind zielgerichtet übermittelt werden können. Authentifiziert sich das Beatmungsgerät beispielsweise bei einer Gegenstelle bzw. wird von der Gegenstelle authentifiziert, kann über die eindeutige Identifikation auch nachgeprüft werden, ob Daten für das Beatmungsgerät vorliegen und übertragen werden sollen.

Alternativ oder ergänzend kann das erfinderische Verfahren auch genutzt werden, um eine Autorisierung zwischen dem Benutzer des Beatmungsgeräts und einer Gegenstelle auf Basis eines "Challenge-Response"-Verfahrens zu realisieren. Beispielsweise nutzt der Benutzer eine Gegenstelle, etwa ein Smartphone mit App, zusammen mit dem Beatmungsgerät und möchte sich über die App auch mit einer anderen Gegenstelle, beispielsweise einem Server und/oder einer Fernbetreuung, verbinden. Das Zertifikat des Beatmungsgerätes kann so also auch für die Authentifizierung des Nutzers des Beatmungsgerätes dienen. Durch die Signierung und/oder Verschlüsselung von einer von der Gegenstelle geforderten "Challenge", kann bei der Gegenstelle sichergestellt werden, dass der Nutzer der Gegenstelle in Besitz eines einzigen Beatmungsgerätes - z.B. mit einer bestimmten Seriennummer (Identifikationsnummer) - ist und ihm der Zugang zu bestimmten Funktionen und/oder Privilegien gewährt werden kann. Beispielsweise kann so auch sichergestellt werden, dass Benachrichtigungen zwischen Nutzer und Betreuer sicher übermittelt werden.

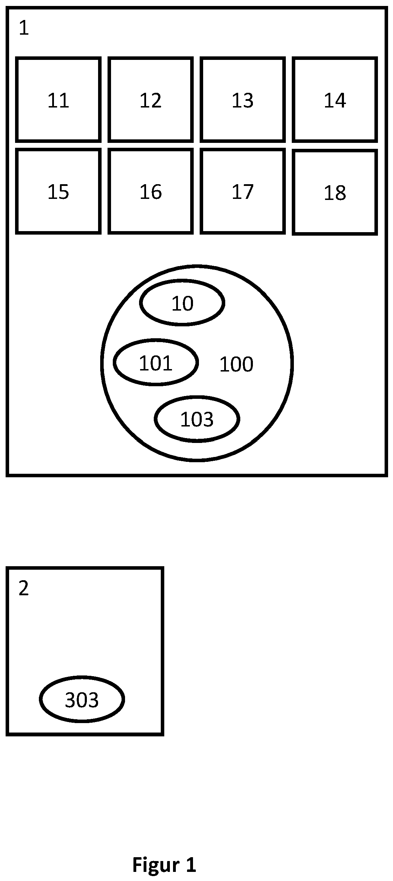

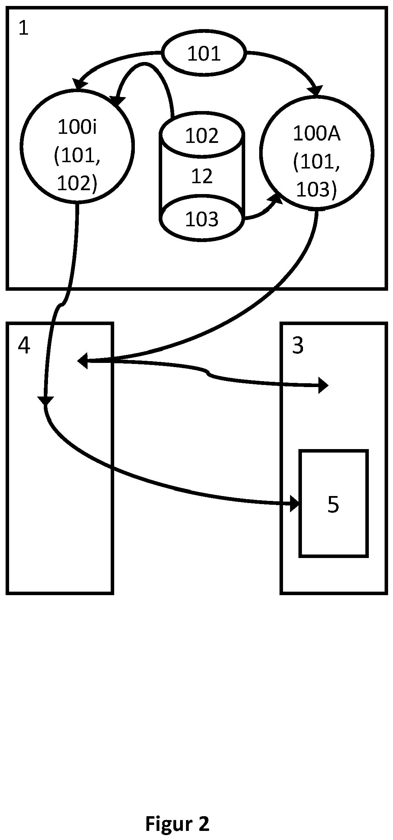

In Figuren 1 bis 7 wird das erfinderische Verfahren zur Authentifizierung anhand Ausführungsbeispielen näher erläutert. Bei den Figuren 2 bis 7 wurden aus Gründen der Übersicht Bestandteile des Beatmungsgerätes sowie Einrichtungen/Einheiten nicht weiter aufgeführt. Das Vorhandensein der Bestandteile, welche zumindest Standardmäßig einem Beatmungsgerät bzw. der entsprechenden Gegenstellen zuzuordnen sind und zum Ablauf des Verfahrens nötig sein können, wie zum Beispiel entsprechende Schnittstellen, wird hier impliziert.

Die Figur 1 zeigt beispielhaft ein Beatmungsgerät 1 mit einer Schnittstelle 11, der Hardwarekomponenten 12, beispielsweise ein Mainboard, eine Anzeigeeinrichtung 13, eine Sensoreinheit 14, eine Steuereinheit 15, eine Gebläse-Nentileinheit 16, eine Aufbereitungseinheit 17 sowie eine Speichereinheit 18. Auf dem Beatmungsgerät ist zudem eine Authentifizierungsdatei 100 gespeichert, welche einen Signaturcode 10, z.B. ein Hashwert, enthält. Weiter ist eine Gegenstelle 2 dargestellt, welcher zumindest der öffentliche Schlüsselcode 303 einer in Figur 1 nicht eingezeichneten Signiereinrichtung 3 bekannt ist. Die Gegenstelle 2 kann dabei ein Server, eine App, ein Programm, ein Computer, ein Smartphone und/oder weitere Geräte sein, welche ausgebildet sind, mit dem Beatmungsgerät 1 eine Verbindung aufzubauen.

Die Sensoreinheit 14 ist eingerichtet, Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann die Sensoreinheit 14 zusätzliche Messungen von Bestandteilen oder Temperatur des Atemgases oder des Blutes vornehmen. Die Sensoreinheit 14 übermittelt die erfassten Messwerte an das Aufbereitungseinheit 17.

Das Aufbereitungseinheit 17 kann die erfassten Messwerte aufbereiten. Beispielsweise kann das Aufbereitungseinheit 17 eine Glättung, eine Artefaktbereinigung oder ein Downsampling der Messwerte durchführen. Beispielsweise ist die Aufbereitungseinheit 17 als kombinierte Aufbereitungs-, Berechnungs- und Erkennungseinheit ausgeführt. Die vorgenannten Einheiten können beispielsweise aber auch als einzelne Einheiten ausgeführt sein. Die Berechnungseinheit berechnet aus den durch die Sensoreinheit 14 erfassten und durch die Aufbereitungseinheit 17 aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten. Die Erkennungseinheit ist eingerichtet, Ereignisse/Zustände wie beispielsweise Alarme, Atemaussetzer, Artefakte, Hustenstöße, Sauerstoff(ent)sättigungen, asynchrone zwischen Gerät und Anwender, Einatmen, Ausatmen oder mandatorische Atemzüge zu erkennen.

Die Speichereinheit 18 speichert unter anderem die durch die Sensoreinheit 14 erfassten Werte/Parameter und/oder die durch die Aufbereitungseinheit 17 und/oder die Berechnungseinheit aufbereiteten Werte, Daten und/oder Informationen, beziehungsweise speichert diese zumindest zwischen. Auch die durch die Erkennungseinheit gewonnenen Informationen, Daten und Werte können und/oder werden in der Speichereinheit zumindest zwischengespeichert. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden.

Die Steuereinheit 15 dient beispielsweise zur Steuerung des Beatmungsgerätes 1, insbesondere einer Gebläse- und/oder Ventileinheit 16 zur Erzeugung des Atemgasstroms bzw. Beatmungsdruckes. Auch kann die Steuereinheit 15 dazu ausgebildet sein, andere Bestandteile und/oder Einheiten des Beatmungsgerätes 1 zu steuern. In manchen Ausführungsformen kann die Steuereinheit 15 auch weiter unterteilt sein und aus mehreren Steuereinheiten bestehen, welche jeweils eine individuelle Einheit und/oder Bestandteil des Beatmungsgerätes 1 steuern.

Die Schnittstelle 11 ist beispielsweise dazu eingerichtet, eine Netzwerkverbindung zu der Gegenstelle 2 aufzubauen. Hierzu kann die Schnittstelle 11 als kabelgebundene oder kabellose Schnittstelle ausgebildet sein. Das Beatmungsgerät 1 kann zudem in manchen Ausführungsformen über eine Vielzahl an Schnittstellen verfügen. Unter anderem können die Schnittstellen aus den folgenden Gruppen ausgewählt sein:
a. drahtlose Schnittstelle für große Entfernungen (Mobilfunk, LPWAN, WLAN) zum Beispiel für Telemonitoring, Teleservice, Telesetting, FW-Update
b. drahtlose Schnittstelle für kleine Entfernungen (Bluetooth, WPAN) zum Beispiel für Fernbedienung über App, Feedback zur Therapie in App, Service-Prozesse; Verbindung zu Diagnose-Geräten (Polygraphie), FW-Update/Firmware-Aktualisierung)
c. Benutzerschnittstelle (User-Interface, GUI, Touch-Screen) zum Beispiel für Bedienung, Feedback, Service
d. Kabel-gebundene Schnittstellen zum Beispiel für Fernsteuerung im Schlaflabor, Auslesen detaillierter Therapiedaten, Verbindung zu Diagnose-Geräten (PSG), externer Anschluss eines Funkmoduls, Service, Kommunikation mit dem Prüfstand, FW-Update
e. Speicher-Medium/Anschluss für USB-Stick, SD-Karte, etc.

Die beispielhaft als Mainboard (Hauptplatine) ausgeführte Hardwarekomponente 12 dient unter anderem als zentrales Elektronikbauteil, mit dem weitere Elektronikbauteile verbunden sind. Beispielsweise sind zumindest die Speichereinheit 18, die Schnittstelle 11, die Steuereinheit 15 sowie die nicht abgebildeten Komponenten wie Prozessor, Arbeitsspeicher (RAM), BIOS-Chip, etc. mit dem Mainboard 12 verbunden und/oder zum Teil auf dem Mainboard verbaut.

Die Anzeigeeinrichtung 13 kann beispielsweise Werte, Daten und Informationen des Beatmungsgerätes 1 anzeigen und wiedergeben. In manchen Ausführungsformen kann die Anzeigeeinrichtung 13 als berührungsempfindlicher Bildschirm (Touch-Screen) ausgeführt sein, über den neben der Anzeige und Wiedergabe auch die Eingabe von Daten und Informationen in das Beatmungsgerät 1 möglich ist.

Auf dem Beatmungsgerät 1 ist eine Authentifizierungsdatei 100 gespeichert, welche beispielhaft einen Signaturcode 10, die Identifikationsnummer 101 des Beatmungsgerätes 1 sowie einen öffentlichen Schlüsselcode 103 beinhaltet. Die Identifikationsnummer 101 kann beispielsweise die Seriennummer des Beatmungsgerätes 1 sein, welche während der Herstellung des Beatmungsgerätes vergeben wird. Der öffentliche Schlüsselcode 103 wird beispielsweise vom Mainboard 12 des Beatmungsgerätes 1 zusammen mit dem privaten Schlüsselcode 103' erstellt, beispielsweise während der Aktivierung/Initiierung des Mainboards 12 während beziehungsweise nach der Herstellung des Beatmungsgerätes 1. Die Schlüsselcodes 103, 103' werden dabei beispielsweise über gängige Generierungsmethoden, welche im Bereich der asymmetrischen Kryptographie bekannt sind, generiert. Der Signaturcode 10 wurde von einer in Figur 1 nicht dargestellten Signiereinrichtung 3 durch Verwendung des geheimen Schlüsselcodes 303' der Signiereinrichtung 3 generiert und während der Produktion des Beatmungsgerätes 1 der Authentifizierungsdatei 100 hinzugefügt. Die Signiereinrichtung 3 stellt dabei eine vertrauenswürdige Einrichtung dar, über welche somit bestätigt werden kann, dass die Authentifizierungsdatei 100 echt ist und auch der Inhalt der Authentifizierungsdatei 100 damit vertrauenswürdig ist. Die Authentifizierungsdatei 100 stellt also ein Zertifikat unter anderem über die Identität des Beatmungsgerätes 1 dar, welches von einer dritten, vertrauenswürdigen Stelle überprüft und signiert wird. Vertrauenswürdige Signiereinrichtungen (auch "Certification Authority", kurz CA, genannt) können dabei staatliche, behördliche oder auch kommerzielle Einrichtungen sein, beispielsweise auch der Hersteller des Beatmungsgerätes 1, welcher zum Beispiel eine Signiereinrichtung 3 betreibt um entsprechende Authentifizierungsdateien 100 der von ihm hergestellten Beatmungsgeräte 1 entsprechend zu signieren.

Der Signaturcode 10 kann beispielsweise über einen öffentlichen Schlüsselcode 303 der Signiereinrichtung 3 überprüft werden. Damit kann beispielsweise eine Gegenstelle 2, soweit der Gegenstelle 2 der öffentliche Schlüsselcode 303 bekannt ist, überprüfen, ob die Authentifizierungsdatei 100 vom Beatmungsgerät 1 vertrauenswürdig ist, also, dass die Authentifizierungsdatei 100 überprüft und schließlich von der vertrauenswürdigen Signiereinrichtung 3 mit dem Signaturcode 10 versehen wurde.

Bei Verbindungsaufbau zwischen Beatmungsgerät 1 und der Gegenstelle 2 wird gemäß einem Standardprotokoll für den Aufbau von Verbindungen, beispielsweise ein SSL-Protokoll oder TLS-Protokoll, die Authentifizierungsdatei 100 von der Gegenstelle 2 überprüft. Erkennt die Gegenstelle 2 den Signaturcode 10 als von der Signiereinrichtung 3 stammend, so wird die Authentifizierungsdatei 100 als vertrauenswürdig akzeptiert, das Beatmungsgerät 1 also authentifiziert und die Verbindung mit den Beatmungsgerät 1 akzeptiert und aufgebaut.

Die Signiereinrichtung 3 kann beispielsweise ein einzelner Computer oder Server sein, in manchen Ausführungsformen ist unter der Signiereinrichtung 3 auch ein System aus mehreren Computern und/oder Servern zu verstehen. Insbesondere kann die Signiereinrichtung 3 beispielsweise auch ein PKI-Service (z.B. zumindest bestehend aus Certification Authority und Request Authority) sein.

Die Gegenstelle 2 umfasst beispielsweise eine Anwenderdatenbank oder ist mit einer Anwenderdatenbank verbunden. Verbindet sich das Beatmungsgerät 1 mit der Gegenstelle 2, kann die Gegenstelle 2 zum Beispiel anhand einer Identifikationsnummer 101 des Beatmungsgerätes 1, welche in der Authentifizierungsdatei 100 enthalten ist, das Beatmungsgerät 1 einem Anwender zuordnen. Durch die vorherige Authentifizierung des Beatmungsgerätes 1 an der Gegenstelle 2 kann so beispielsweise sichergestellt werden, dass die übertragenen Daten wirklich vom Beatmungsgerät 1 stammen.

In manchen Ausführungsformen umfasst die Gegenstelle 2, beispielsweise als eine einfache Smartphone-App zum Auslesen der Daten des Beatmungsgerätes 1 ausgeführt, keine Anwenderdatenbank. Dennoch ist hier eine Authentifizierung zum Verbindungsaufbau nötig.

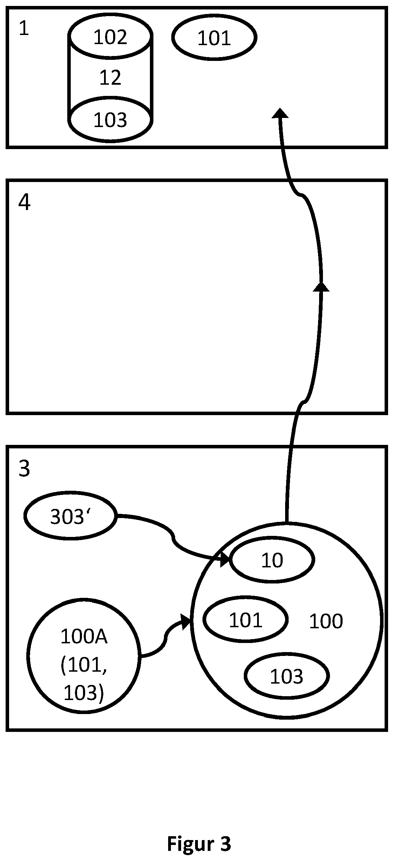

In Figuren 2 und 3 ist beispielhaft dargestellt, wie die Authentifizierungsdatei 100 bei der Herstellung des Beatmungsgerätes 1 erzeugt wird. Bei der ersten Initialisierung/Aktivierung des Beatmungsgerätes 1 beziehungsweise des Mainboard 12 wird ein Paar von Schlüsselcodes 103, 103' erstellt, welche direkt mit dem Mainboard 12 assoziiert sind bzw. direkt auf dem Mainboard 12 gespeichert werden. Bei der ersten Aktivierung wird zudem die Identifikationsnummer 101 des Beatmungsgerätes 1 über eine Benutzerschnittstelle (z.B. eine als Touch-Screen ausgeführte Anzeigeeinrichtung 13, eine Speicherkarte, eine Smartphone-App, etc.) eingegeben und dem Beatmungsgerät 1 zur Verfügung gestellt. Das Beatmungsgerät 1 erstellt daraufhin zwei Dateien 100A und 100i: eine Anfragedatei 100A, mit welcher die Authentifizierungsdatei 100 generiert wird, sowie die Informationsdatei 100i, welche Informationen über das Beatmungsgerät 1 enthält. Beide Dateien 100A, 100i werden auf eine Prüfeinrichtung 4, welche beispielsweise primär zur Überprüfung auf Funktionsfähigkeit mit dem Beatmungsgerät 1 verbunden wird, übertragen. Die Informationsdatei 100i enthält dabei zumindest die Identifikationsnummer 101 des Beatmungsgerätes 1 sowie die Identifikationsnummer 102 des Mainboards 12. Die Anfragedatei 100A enthält zumindest den generierten öffentlichen Schlüsselcode 103 sowie die Identifikationsnummer 101 des Beatmungsgerätes 1. Auf der Signiereinrichtung 3 ist zudem eine Liste 5 gespeichert, welche gültige und/oder bekannte Kombinationen von Beatmungsgeräten 1 und Mainboards 12 und/oder deren Identifikationsnummern 1, 12 umfasst. In manchen beispielhaften Ausführungsformen ist die Liste 5 nicht auf der Signiereinrichtung 3 gespeichert, sondern in einer Datenbank oder auf einem Datenserver hinterlegt, auf welche die Signiereinrichtung 3 Zugriff hat. Anhand der Liste 5 wird überprüft, ob es sich bei der Kombination des Beatmungsgerätes 1 mit dem Mainboard 12 um eine bereits vorhandene und eine gültige beziehungsweise freigegebene Kombination handelt. Ist die Kombination noch nicht vorhanden aber gültig/freigegeben, so wird die Kombination 101 mit 102 bzw. 1 mit 12 in die Liste 5 eingetragen, wie in Figur 3 schematisch angedeutet. Soweit es sich bei der Kombination um eine gültige Kombination handelt, wird mit der Anfragedatei 100A über die Signierstelle 3 die Authentifizierungsdatei 100 erstellt.

In Figur 3 ist weiter schematisch angedeutet, dass die Signiereinrichtung 3 aus der Anfragedatei 100A die Authentifizierungsdatei 100 erstellt und dieser unter Verwendung des geheimen Schlüsselcodes 303' den Signaturcode 10 hinzufügt. Die Erstellung des Signaturcodes 10 erfolgt dabei so, dass mit dem öffentlichen Schlüsselcode 303 der Signiereinrichtung der Signaturcode 10 zumindest auf die Herkunft von Signiereinrichtung 3 überprüft werden kann. In manchen Ausführungsformen ist der Signaturcode 10 darüber hinaus auf die Authentifizierungsdatei 100 zugeschnitten, passt also nur zu dieser Authentifizierungsdatei. Nach Erstellung der Authentifizierungsdatei 100 wird diese an die Prüfeinrichtung 4 übertragen und von dort auf das Beatmungsgerät 1.

In manchen beispielhaften Ausführungsformen sind die Verfahrensschritte so eingerichtet, dass - soweit möglich - das Verfahren vollautomatisch abläuft. So wäre beispielsweise lediglich die Eingabe der Identifikationsnummer 101 am Beatmungsgerät 1 manuell zu tätigen, da diese zum Zeitpunkt der ersten Aktivierung noch nicht elektronisch im Beatmungsgerät 1 vorliegt. In manchen Ausführungsformen kann es beispielsweise auch so vorgesehen sein, dass einzelne Schritte manuell zumindest angestoßen und/oder durchgeführt werden müssen. Beispielsweise ist das Übertragen der Anfragedatei 100A auf die Signiereinrichtung 3 zu nennen, welches in manchen Ausführungsformen zunächst einer manuellen Bestätigung an der Prüfeinrichtung 4 bedarf. Es ist zum Beispiel zudem auch denkbar, dass die Überprüfung der Liste 5 auf vorhandene, gültige und freigegebene Kombinationen von Beatmungsgerät 1 und Mainboard 12 manuell durchgeführt wird. In manchen beispielhaften Ausführungsformen werden mehrere oder alle Schritte manuell angestoßen und/oder durchgeführt.

Die Erstellung des Signiercodes 10 durch die Signiereinrichtung 3 unter Verwendung des geheimen Schlüsselcodes 303' erfolgt dabei beispielsweise unter Berücksichtigung bekannter und/oder gängiger Verfahren aus dem Bereich der Private/Public Key Infrastructure (PKI).

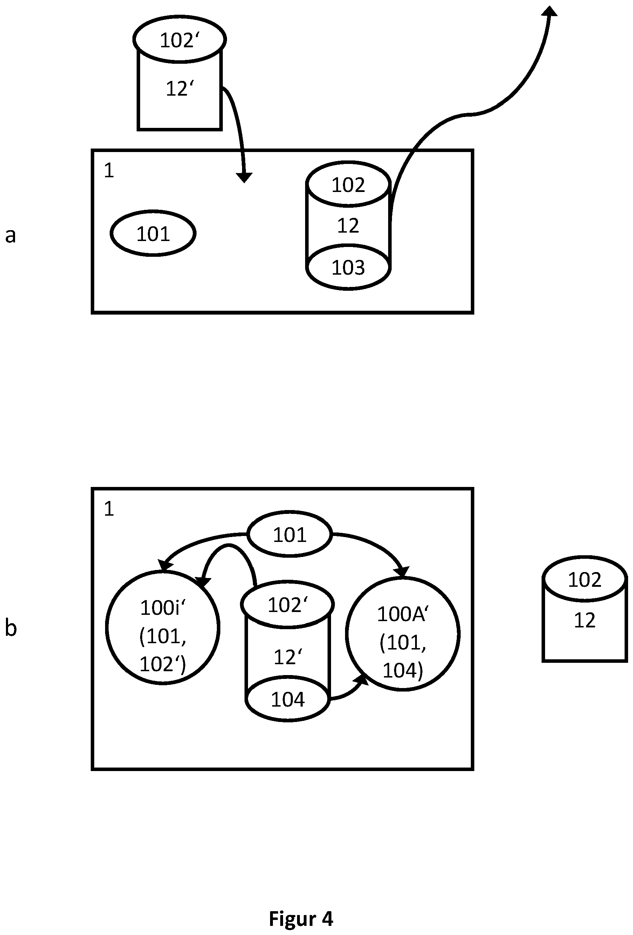

Wird die Hardwarekomponente/das Mainboard 12 gegen eine neue Hardwarekomponente/ein neuen Mainboard 12' ausgetauscht, so muss auch die Authentifizierungsdatei 100 inklusive des Paars von Schlüsselcodes 103, 103' erneuert werden, welche beispielsweise direkt auf dem Mainboard 12 hinterlegt sind. Die Schritte des Austauschs sind beispielhaft in den Figuren 4a, 4b, 5, 6 dargestellt. In Figur 4a ist beispielhaft zu sehen, dass mit Austausch des Mainboards 12 auch die Identifikationsnummer 102 des Mainboards 12 sowie die Schlüsselcodes 103, 103' (letzterer nicht dargestellt) entfernt werden. In manchen Ausführungsformen ist zudem auch die Authentifizierungsdatei 100 auf dem Mainboard 12 gespeichert, welche mit Austausch ebenfalls verloren geht. Darüber hinaus wird die Authentifizierungsdatei 100 ebenfalls ungültig, da diese auf eine Kombination von zumindest dem öffentlichen Schlüssel 103 und der Identifikationsnummer 101 erstellt ist. Bei der ersten Aktivierung des neuen Mainboards 12', zum Beispiel während der Produktion als Austauschkomponente oder nach Einbau im Beatmungsgerät 1, wird erneut ein öffentlicher Schlüsselcode 104 sowie ein geheimer Schlüsselcode 104' vom Mainboard 12' generiert. In manchen Ausführungsformen wird das Mainboard 12' bereits beim Hersteller aktiviert und/oder initiiert, wobei die Schlüsselcodes 104, 104'erstellt werden. Beispielsweise muss in manchen Ausführungsformen bei oder nach der Aktivierung des Mainboards 12' die Identifikationsnummer 101 des Beatmungsgerätes 1 eingegeben werden. Während und/oder nach der Aktivierung des Mainboards 12 werden auch die Informationsdatei 100i', welche zumindest die Identifikationsnummern 101, 102' enthält, sowie die Anfragedatei 100A', umfassend zumindest den öffentlichen Schlüsselcode 104 und die Identifikationsnummer 101, erstellt.

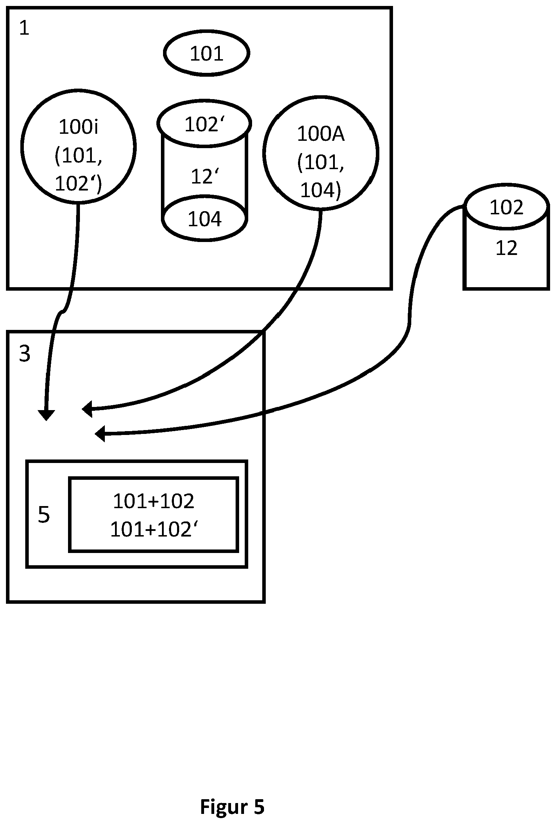

In einem weiteren Schritt, beispielhaft in Figur 5 dargestellt, werden die Dateien 100i', 100A' sowie die Identifikationsnummer 102 des alten Mainboards 12 an die Signiereinrichtung 3 übermittelt. Die Signiereinrichtung 3 prüft daraufhin, ob die alte Kombination des Beatmungsgerätes 1 mit dem Mainboard 12 eine gültige Kombination war, beispielsweise anhand der Identifikationsnummern 101, 102. Die Identifikationsnummer 102 ist beispielsweise nicht nur elektronisch auf dem Mainboard 12 hinterlegt, sondern ebenfalls auf das Bauteil aufgedruckt/aufgeklebt. Die gültigen Kombinationen sind zum Beispiel in der Liste 5 hinterlegt, welche zum Beispiel direkt auf der Signiereinrichtung 3 gespeichert ist, aber beispielsweise auch in einer Datenbank oder einem Server hinterlegt bzw. gespeichert sein kann. Auch wird, beispielsweise bei Anforderung eines neues Mainboards 12' durch einen Service-Techniker oder einen Händler, hinterlegt, dass das Mainboard 12' für eine Kombination mit einem Beatmungsgerät freigegeben wird. Beispielsweise wird dazu die neue Kombination von 101 und 102' als freigegebene Kombination in die Liste 5 eingetragen. Für manche Ausführungsformen ist es auch denkbar, dass das Mainboard 12' allgemein für die Kombination mit einem Beatmungsgerät freigegeben wird. Werden beispielsweise von einem Händler oder Service-Techniker mehrere neue Mainboards angefordert, können diese "frei" mit den Beatmungsgeräten kombiniert werden, ohne dass der Techniker genau darauf achten muss, welches Beatmungsgerät mit welchem Mainboard kombiniert wird. Beispielsweise kann bei der Anforderung (Bestellung) der neuen Mainboards auch bereits die Identifikationsnummern der Beatmungsgeräte angefragt werden, welche ein neues Mainboard erhalten sollen. So können die neuen Mainboards für nur genau diese Beatmungsgeräte freigegeben werden. Eine Kombination mit anderen Beatmungsgeräten würde bei Prüfung der Liste 5 als nicht-gültig erkannt werden und entsprechend keine Authentifizierungsdatei für ebenjene Kombination erstellt werden. Die Prüfung, ob das neue Mainboard 12' mit dem Beatmungsgerät 1 kombiniert werden darf beziehungsweise eine Authentifizierungsdatei 100' erstellt wird, kann beispielsweise automatisch durch die Signiereinrichtung 3unter Verwendung der Identifikationsnummern 101, 102, 102' stattfinden. In manchen Ausführungsformen des Verfahrens kann auch eine manuelle Prüfung der Kombinationen stattfinden.

In manchen Ausführungsformen des Verfahrens sind die Informationen über gültige Kombinationen von Beatmungsgeräten und Mainboards sowie allgemein Daten wie der öffentliche Schlüsselcode und die Identifikationsnummer des Mainboards auf der Signiereinrichtung 3 gespeichert oder zumindest für die Signiereinrichtung 3 zugänglich.

Beispielsweise wird bereits bei der Produktion des neuen Mainboards 12', welches als Austauschteil vorgesehen ist, dieses initialisiert. Das bedeutet, dass das Mainboard 12' bereits zu dem Zeitpunkt zumindest den öffentlichen Schlüsselcode 104 generiert und zudem Informationen über zumindest die Identifikationsnummer 102' sowie den Schlüsselcode 104 an die Signiereinrichtung 3 übermittelt bzw. diese Informationen übermittelt werden. Dadurch kann die Signiereinrichtung 3 zu einem späteren Zeitpunkt überprüfen, ob das neue Mainboard 12' für den Einsatz in einem Beatmungsgerät vorgesehen ist.

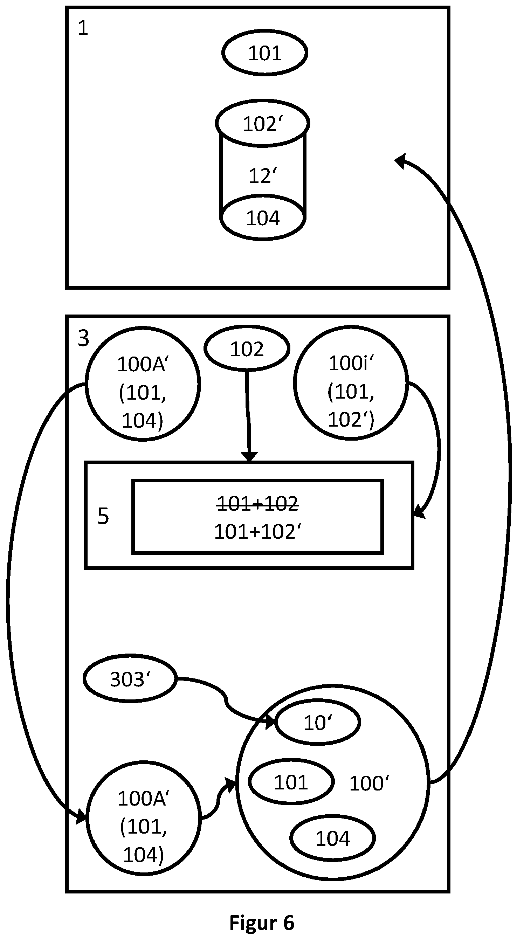

Wird die Kombination des Beatmungsgerätes 1 mit dem Mainboard 12' als freigegeben bzw. gültig erkannt, so wird die Anfragedatei 100A' auf die Signiereinrichtung 3 übertragen. Die Signiereinrichtung 3 erstellt daraufhin aus der Anfragedatei 100A' die Authentifizierungsdatei 100' und fügt dieser Authentifizierungsdatei 100' den Signaturcode 10' unter Verwendung des geheimen Schlüsselcodes 303' hinzu. Die Authentifizierungsdatei 100' kann dann beispielsweise gemäß Figur 6 direkt auf das Beatmungsgerät 1 übertragen werden.

Weiter wird die alte Kombination von Beatmungsgerät 1 und Mainboard 12 bzw. der entsprechenden Identifikationsnummern 101, 102 beispielsweise aus der Liste 5 gelöscht und/oder als veraltet und ungültig markiert. In manchen Ausführungsformen kann es zudem auch eine weitere Liste und/oder Datenbank geben, in der veraltete und/oder ungültige Authentifizierungsdateien vermerkt werden. Darüber hinaus ist es denkbar, dass zusätzlich eine weitere Liste/Datenbank geführt wird, in welcher bekannte, ungültige Kombinationen vermerkt werden. Insbesondere eine Liste mit veralteten und/oder ungültigen Authentifizierungsdateien kann zum Beispiel der Gegenstelle 2 zur Verfügung gestellt werden, sodass die Gegenstelle 2 zusätzlich prüfen kann, ob die vom Beatmungsgerät 1 gesendete Authentifizierungsdatei 100, 100' aktuell gültig ist.

In manchen Ausführungsformen wird zur Erstellung einer neuen Authentifizierungsdatei 100' keine Informationsdatei 100i' erstellt und/oder an die Signiereinrichtung 3 übertragen, sondern die nötigen Daten werden einem Mitarbeiter des Herstellers bzw. Betreibers der Signiereinrichtung 3 mitgeteilt, welcher die Änderungen an Liste 5 und/oder zusätzlichen Listen/Datenbanken manuell vornimmt. Auch ist es möglich, direkt vom Beatmungsgerät 1 auf die Signiereinrichtung 3, beispielsweise über einen Datenträger, oder über andere Wege übertragen wird.

Verbindet sich ein Beatmungsgerät 1 mit der Gegenstelle 2 und sendet zum Beispiel eine Authentifizierungsdatei 100, basierend auf der Kombination des

Beatmungsgerätes 1 mit dem Mainboard 12, obwohl die Kombination aus 1 und 12 keine aktuell gültige Kombination ist, so kann die Gegenstelle 2 die Verbindung mit dem Beatmungsgerät 1 nicht akzeptieren, es findet also keine Verbindung statt. In manchen Ausführungsformen sendet die Gegenstelle 2 in dem Fall einer ungültigen Authentifizierung des Beatmungsgerätes 1 eine entsprechende Meldung zumindest an das Beatmungsgerät 1. Diese Meldung kann verschiedene Informationen enthalten, beispielsweise einen Hinweis darauf, dass die Authentifizierungsdatei 100 ungültig ist und/oder einen Hinweis, dass eine Service-Hotline kontaktiert werden soll. Beispielsweise kann zudem bei Verwendung einer ungültigen Authentifizierungsdatei 100 auch ein Befehl an das Beatmungsgerät 1 gesendet werden, welcher eine Sperrung des Beatmungsgerätes 1 zur Folge hat, welche mit einer entsprechenden Meldung auf der Anzeigeeinrichtung 13 des Beatmungsgerätes 1 einhergeht.

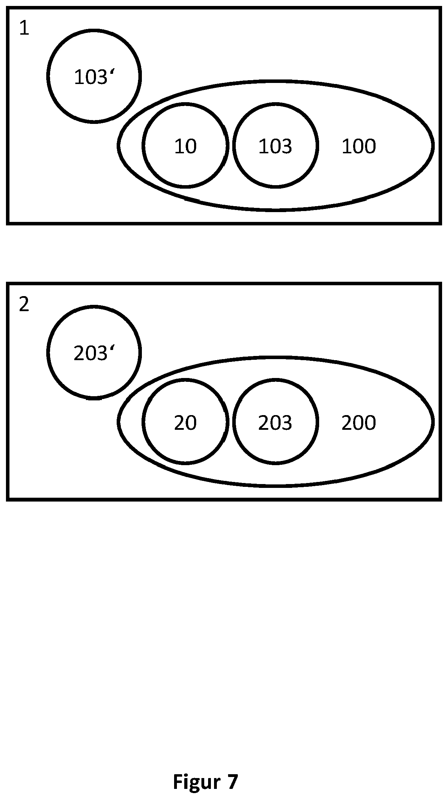

In manchen Ausführungsformen ist auch auf der Gegenstelle 2 eine Authentifizierungsdatei 200 gespeichert, welche ebenfalls einen durch die Signierstelle 3 erzeugten Signaturcode 20 enthält, wie in Figur 7 schematisch dargestellt. Das Beatmungsgerät 1 kann, wie auch andere Gegenstellen, bei Kenntnis des öffentlichen Schlüsselcodes 303 der Signiereinrichtung 3 (nicht dargestellt), überprüfen, ob der Signaturcode 20 tatsächlich von der Signierstelle 3 erstellt wurde und somit die Gegenstelle 2 authentifizieren. Zudem enthält die Authentifizierungsdatei 200 einen öffentlichen Schlüsselcode 203. Die Authentifizierungsdatei 200 kann unter anderem bei einer Verbindung mit dem Beatmungsgerät 1 übertragen werden. Sollen Daten vom Beatmungsgerät 1 an die Gegenstelle 2 übertragen werden, so können diese beispielsweise mit dem öffentlichen Schlüsselcode 203 der Gegenstelle 2 verschlüsselt werden. Daten, welche mit dem öffentlichen Schlüsselcode 203 verschlüsselt wurden können beispielsweise nur mit dem geheimen Schlüsselcode 203' wieder entschlüsselt werden. Andersherum kann die Gegenstelle 2 auch den öffentlichen Schlüsselcode 103 des Beatmungsgerätes 1 nutzen um Daten zu verschlüsseln, welche nur durch den geheimen Schlüsselcode 103' wieder entschlüsselt werden können.

Dies kann beispielsweise, aber nicht ausschließlich, Konfigurationsdaten für das Beatmungsgerät 1 betreffen. Es kann beispielsweise vorgesehen sein, dass die Gegenstelle 2 die Konfigurationsdaten mit dem Signaturcode 20 signiert. Das Beatmungsgerät 1 kann, beispielweise über den öffentlichen Schlüsselcode 303 der Signiereinrichtung 3, die Konfigurationsdaten authentifizieren. Bei erfolgreicher Authentifizierung können die Konfigurationsdaten dann übernommen bzw. angewendet werden. Findet die Übertragung der Konfigurationsdaten beispielsweise von einer entfernten Gegenstelle 2 auf das Beatmungsgerät 1 statt, kann vorgesehen sein, dass sich das Beatmungsgerät 1 zunächst bei der Gegenstelle 2 authentifizieren muss. Somit kann auch sichergestellt werden, dass die Konfigurationsdaten nur auf das dafür vorgesehene Beatmungsgerät 1 übertragen werden.

Fernen kann vorgesehen sein, dass die Konfigurationsdaten mit dem öffentlichen Schlüssel 103 des Beatmungsgeräts 1 verschlüsselt werden, sodass diese ausschließlich mit dem geheimen Schlüssel 103' des Beatmungsgerätes 1 entschlüsselt werden können. Die übermittelten Konfigurationsdaten können entsprechend nur vom Beatmungsgerät 1 verwendet werden.

### Bezugszeichenliste

- 1: Beatmungsgerät
- 2: Gegenstelle
- 3: Signiereinrichtung
- 4: Prüfeinrichtung
- 5: Liste
- 10: Signaturcode
- 10': Signaturcode
- 11: Schnittstelle
- 12: Hardwarekomponente/Mainboard
- 12': Hardwarekomponente/Mainboard
- 13: Anzeigeeinrichtung
- 14: Sensoreinheit
- 15: Steuereinheit
- 16: Gebläse-/Ventileinheit
- 17: Aufbereitungseinheit
- 18: Speichereinheit
- 20: Signaturcode
- 100: Authentifizierungsdatei
- 100': Authentifizierungsdatei
- 100A: Anfragedatei
- 100A': Anfragedatei
- 100i: Informationsdatei
- 100i': Informationsdatei
- 101: Identifikationsnummer
- 102: Identifikationsnummer
- 102': Identifikationsnummer
- 103: öffentlicher Schlüsselcode
- 103': geheimer Schlüsselcode
- 104: öffentlicher Schlüsselcode
- 104': geheimer Schlüsselcode
- 200: Authentifizierungsdatei
- 203: öffentlicher Schlüsselcode
- 203': öffentlicher Schlüsselcode
- 303: öffentlicher Schlüsselcode
- 303': geheimer Schlüsselcode

## Patentansprüche

1. Verfahren zur Authentifizierung zumindest eines Beatmungsgerätes (1) bei zumindest einer Gegenstelle (2), wobei sich das Beatmungsgerät (1) über zumindest eine Schnittstelle (11) mit der Gegenstelle (2) verbinden kann, wobei auf dem Beatmungsgerät (1) mindestens eine Authentifizierungsdatei (100) gespeichert ist, wobei die Authentifizierungsdatei (100) zumindest einen Signaturcode (10) einer Signiereinrichtung (3) beinhaltet und der Gegenstelle (2) ein öffentlicher Schlüsselcode (303) der Signiereinrichtung (3) bekannt ist **dadurch gekennzeichnet, dass** das Beatmungsgerät (1) bei Verbindungsaufbau mit der Gegenstelle (2) die Authentifizierungsdatei (100) an die Gegenstelle (2) sendet, wobei die Gegenstelle (2) mit dem öffentlichen Schlüsselcode (303) den Signaturcode (10) der Authentifizierungsdatei (100) daraufhin überprüft, ob der Signaturcode (10) von der Signierstelle (3) stammt und wobei das Beatmungsgerät (1) authentifiziert wird, wenn die Gegenstelle (2) den Signaturcode (10) als von der Signiereinrichtung (3) stammend erkennt.

2. Verfahren nach zumindest einem der vorhergehenden Ansprüche, **dadurch gekennzeichnet, dass** nach und/oder während des Verbindungsaufbaus therapeutische Daten vom Beatmungsgerät (1) auf die Gegenstelle (2) übertragen werden, wobei die therapeutischen Daten von der Gegenstelle (2) nur dann verarbeitet werden, wenn das Beatmungsgerät (1) authentifiziert wird.

3. Verfahren nach zumindest einem der vorhergehenden Ansprüche, **dadurch gekennzeichnet, dass** die Verbindung vom Beatmungsgerät (1) zur Gegenstelle (2) nur dann aufgebaut und/oder akzeptiert und/oder aufrechterhalten wird, wenn die Gegenstelle (2) den Signaturcode (10) als von der Signiereinrichtung (3) stammend erkennt und das Beatmungsgerät (1) authentifiziert ist.

4. Verfahren nach zumindest einem der vorhergehenden Ansprüche, **dadurch gekennzeichnet, dass** vom Beatmungsgerät (1) an die Gegenstelle (2) übertragene therapeutische Daten von der Gegenstelle (2) verworfen werden, wenn die Gegenstelle (2) den Signaturcode (10) nicht als von der Signiereinrichtung (3) stammend erkennt.

5. Verfahren nach zumindest einem der vorhergehenden Ansprüche **dadurch gekennzeichnet, dass** die Signiereinrichtung (3) die Authentifizierungsdatei (100) erstellt und der Authentifizierungsdatei (100) unter Verwendung eines geheimen Schlüssels (303') der Signiereinrichtung (3) den Signaturcode (10) hinzufügt die Authentifizierungsdatei (100) neben dem Signaturcode (10) mindestens eine Identifikationsnummer (101) des Beatmungsgerätes (1) beinhaltet, wobei die Gegenstelle (2) das Beatmungsgerät (1) anhand der Identifikationsnummer (101) erkennt und einem Anwender zuordnet.

6. Verfahren nach zumindest einem der vorhergehenden Ansprüche **dadurch gekennzeichnet, dass** das Beatmungsgerät (1) eine Anfragedatei (100A) zur Anfrage der Erstellung einer Authentifizierungsdatei (100) durch die Signiereinrichtung (3) erzeugt, wobei die Anfragedatei (100A) mindestens die Identifikationsnummer (101) des Beatmungsgerätes (1) enthält.

7. Verfahren nach zumindest einem der vorhergehenden Ansprüche **dadurch gekennzeichnet, dass** das Beatmungsgerät (1) neben der Anfragedatei (100A) eine Informationsdatei (100i) erstellt, welche mindestens die Identifikationsnummer (101) des Beatmungsgerätes (1) und mindestens eine Identifikationsnummer (102) mindestens einer Hardwarekomponente (12) des Beatmungsgerätes (1) enthält, wobei die Anfragedatei (100A) und die Informationsdatei (100i) auf eine Prüfeinrichtung (4) übertragen werden, die in der Informationsdatei (100i) enthaltene Kombination der Identifikationsnummern (101, 102) auf Gültigkeit überprüft wird und wenn die Kombination der Identifikationsnummern (101, 102) gültig ist, die Prüfeinrichtung (4) die Anfragedatei (100A) an die Signiereinrichtung (3) weiterleitet und die Signiereinrichtung (3) unter Verwendung der Anfragedatei (100A) die Authentifizierungsdatei (100) erzeugt.

8. Verfahren nach zumindest einem der vorhergehenden Ansprüche **dadurch gekennzeichnet, dass** die übertragene Kombination der Identifikationsnummern (101, 102) in eine Datenbank eingetragen werden, falls mit der Identifikationsnummer (101) des Beatmungsgerätes (1) noch keine Kombination mit einer Identifikationsnummer einer Hardwarekomponente eingetragen ist und falls eine Kombination der Identifikationsnummer (101) des Beatmungsgerätes (1) mit der Identifikationsnummer einer Hardwarekomponente eingetragen ist, welche nicht der übertragenen Kombination der Identifikationsnummern entspricht, die übertragene Kombination als nicht gültig gewertet wird und die Anfragedatei (100A) nicht an die Signiereinrichtung (3) weitergeleitet wird.

9. Verfahren nach zumindest einem der vorhergehenden Ansprüche **dadurch gekennzeichnet, dass** bei Austausch der Hardwarekomponente (12) eine neue Anfragedatei (100A') vom Beatmungsgerät (1) unter Verwendung zumindest einer Identifikationsnummer (102') der neuen Hardwarekomponente (12') und der Identifikationsnummer (101) des Beatmungsgerätes erstellt wird.

10. Verfahren nach zumindest einem der vorhergehenden Ansprüche **dadurch gekennzeichnet, dass** die neue Kombination von Identifikationsnummer (102') der neuen Hardwarekomponente (12') und Identifikationsnummer (101) des Beatmungsgerätes (1) auf Gültigkeit überprüft wird, wobei bei einer gültigen neuen Kombination der Identifikationsnummern (101, 102') die Anfragedatei (100A') auf die Signiereinrichtung (3) übertragen wird, wobei die Signiereinrichtung (3) eine neue Authentifizierungsdatei (100') erstellt und unter Verwendung des geheimen Schlüsselcodes (303') der neuen Authentifizierungsdatei (100') einen Signaturcode (10') hinzufügt und die neue Authentifizierungsdatei (100') danach auf das Beatmungsgerät (1) übertragen wird.

11. Verfahren nach zumindest einem der vorhergehenden Ansprüche **dadurch gekennzeichnet, dass** die alte Kombination der Identifikationsnummern (101, 102) des Beatmungsgerätes (1) und der alten Hardwarekomponente (12) nach Tausch der alten Hardwarekomponente (12) mit der neuen Hardwarekomponente (12') als ungültig markiert wird und als ungültig markierte Kombinationen von Beatmungsgeräten und Hardwarekomponenten der Gegenstelle (2) zur Verfügung gestellt werden.

12. Verfahren nach zumindest einem der vorhergehenden Ansprüche **dadurch gekennzeichnet, dass** die Gegenstelle (2) zur Verfügung gestellten und als ungültig markierten Kombinationen von Zeit zu Zeit abruft, wobei die Gegenstelle (2) eine Verbindung mit einem Beatmungsgerät (1), welches sich mit einer Authentifizierungsdatei (100) authentifizieren möchte, welche unter Verwendung einer ungültigen Kombination erstellt wurde, nicht akzeptiert, sodass keine Übertragung von therapeutischen Daten zwischen dem Beatmungsgerät (1) und der Gegenstelle (2) stattfindet und/oder die vom Beatmungsgerät (1) an die Gegenstelle (2) gesendeten therapeutischen Daten nicht von der Gegenstelle (2) verarbeitet werden und/oder von der Gegenstelle (2) verworfen werden.

13. Verfahren nach zumindest einem der vorhergehenden Ansprüche **dadurch gekennzeichnet, dass** die Gegenstelle (2) bei einem Authentifizierungsversuch eines Beatmungsgerätes (1) mit einer Authentifizierungsdatei (100) aus einer ungültigen Kombination von Hardwarekomponente und Beatmungsgerät eine Meldung generiert und/oder einen Befehl an das Beatmungsgerät (1) sendet, welcher die Benutzung des Beatmungsgerätes (1) sperrt und eine Servicemeldung auf einer Anzeigeeinrichtung (13) des Beatmungsgerätes (1) anzeigt.

14. Verfahren nach zumindest einem der vorhergehenden Ansprüche **dadurch gekennzeichnet, dass** das von der Hardwarekomponente (12, 12') ein geheimer Schlüsselcode (103') und ein öffentlicher Schlüsselcode (103) für das Beatmungsgerät (1) generiert wird und der öffentliche Schlüsselcode (103) als Teil der Anfragedatei (100A, 100A') an die Signiereinrichtung (3) übermittelt wird, wobei der öffentliche Schlüsselcode (103) zur Verschlüsselung von Daten dient, welche an das Beatmungsgerät (1) gesendet werden und die mit dem öffentlichen Schlüsselcode (103) des Beatmungsgerätes (1) verschlüsselten Daten nur mit den geheimen Schlüsselcode (103') des Beatmungsgerätes (1) entschlüsselt werden können.

15. Verfahren nach zumindest einem der vorhergehenden Ansprüche **dadurch gekennzeichnet, dass** die Gegenstelle (2) über einen geheimen Schlüsselcode (203') und einen öffentlichen Schlüsselcode (203) verfügt, wobei der öffentliche Schlüsselcode (203) als Teil einer Authentifizierungsdatei (200) dem Beatmungsgerät (1) zur Verfügung gestellt wird, wobei das Beatmungsgerät (1) Daten, welche vom Beatmungsgerät (1) an die Gegenstelle (2) übertragen werden, mit dem öffentlichen Schlüsselcode (203) verschlüsseln und wobei die Daten, welche mit dem öffentlichen Schlüsselcode (203) verschlüsselt wurden, nur mit dem geheimen Schlüsselcode (203') entschlüsselt werden können.

16. Verfahren nach zumindest einem der vorhergehenden Ansprüche **dadurch gekennzeichnet, dass** die Authentifizierungsdatei (200) von der Signiereinrichtung (3) erstellt wird und die Signiereinrichtung (3) der Authentifizierungsdatei (200) einen Signaturcode (20) unter Verwendung des geheimen Schlüsselcodes (303') hinzufügt, wobei das Beatmungsgerät (1) den Signaturcode (20) mit dem öffentlichen Schlüsselcode (303) überprüft und die Authentifizierungsdatei (200) nur akzeptiert, wenn der Signaturcode (20) als von der Signiereinrichtung (3) stammend erkannt wird.

17. System umfassend zumindest ein Beatmungsgerät (1) und zumindest eine Gegenstelle (2), wobei das Beatmungsgerät (1) eingerichtet ist über zumindest eine Schnittstelle (11) eine Verbindung mit der Gegenstelle (2) aufzubauen und wobei auf dem Beatmungsgerät (1) zumindest eine Authentifizierungsdatei (100) gespeichert ist, welche zumindest einen Signaturcode (10) einer Signiereinrichtung (3) beinhaltet, wobei der Gegenstelle (2) ein öffentlicher Schlüsselcode (303) der Signiereinrichtung (3) bekannt ist **dadurch gekennzeichnet, dass** das Beatmungsgerät (1) bei Verbindungsaufbau mit der Gegenstelle (2) die Authentifizierungsdatei (100) an die Gegenstelle (2) sendet, wobei die Gegenstelle (2) mit dem öffentlichen Schlüsselcode (303) den Signaturcode (10) der Authentifizierungsdatei (100) daraufhin überprüft, ob der Signaturcode (10) von der Signierstelle (3) stammt und wobei das Beatmungsgerät (1) authentifiziert wird, wenn die Gegenstelle (2) den Signaturcode (10) als von der Signiereinrichtung (3) stammend erkennt.
